(19) Europäisches Patentamt
European Patent Office
Office européen des brevets

(11) Numéro de publication: **0 079 411**
**B1**

(12) **FASCICULE DE BREVET EUROPÉEN**

(45) Date de publication du fascicule du brevet: **04.02.87**

(21) Numéro de dépôt: **81401811.5**

(22) Date de dépôt: **18.11.81**

(51) Int. Cl.[4]: **C 07 D 471/14**, C 07 D 471/22, A 61 K 31/435, A 61 K 31/505, A 61 K 31/55 // (C07D471/14, 221:00, 221:00, 209:00),(C07D471/22, 239:00, 221:00, 221:00, 209:00), (C07D471/22, 243:00, 221:00, 221:00, 209:00)

(54) **Dérivés d'indoloquinolizine, leur procédé de préparation et leur application en thérapeutique.**

(43) Date de publication de la demande: **25.05.83 Bulletin 83/21**

(45) Mention de la délivrance du brevet: **04.02.87 Bulletin 87/6**

(84) Etats contractants désignés: **AT BE CH DE FR GB IT LI LU NL SE**

(56) Documents cité:
**GB-A-1 435 573**
**GB-A-2 027 017**
**US-A-3 962 258**

**JOURNAL OF MEDICINAL CHEMISTRY, vol. 22, no. 12, décembre 1979 WASHINGTON (US) S.S. KLIOZE et al.: "Synthesis and antihypertensive activity of 2-benzamido-1,2,3,4,6,7,12,12b-octahydroindolo (2,3-a) quinolizines" pages 1497-1504**

(73) Titulaire: **Thal, Claude, 15ter, rue des Clos Saint-Marcel, F-92330 Sceaux (FR)**

(72) Inventeur: **Diatta, Luc, 11, Allée des Bathes, F-91940 Les Ulis (FR)**
Inventeur: **Warolin, Christian, 55, Bd Beauséjour, F-75016 Paris (FR)**

(74) Mandataire: **Martin, Jean-Jacques, Cabinet REGIMBEAU 26, Avenue Kléber, F-75116 Paris (FR)**

**EP 0 079 411 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne des dérivés d'indoloquinolizine, leur procédé de préparation et leur application en thérapeutique, notamment dans le domaine des affectations vasculaires.

Des produits possédant dans l'une des positions 1 à 4 sur le cycle indoloquinolizine un groupement

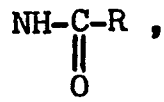

R étant un radical alcoxy ou phényl sont inclus dans la formule générale du brevet anglais 1.435.573. Ces produits sont présentés non comme étant actifs dans le domaine de la circulation cérébrale, mais simplement comme hypotenseurs.

Des indolo (2,3-a) quinolizine possédant en 1 un groupement éthyl et actives dans la circulation sanguine, sont décrites dans le brevet W079/0319.

D'autres dérivés d'indoloquinolizine présentés comme étant utiles dans l'arythmie cardiaque, l'hypertension et l'angine de poitrine, sont revendiqués dans le brevet US 4.187.657. Ces produits portent un groupement benzyle sur l'azote de l'indole et un groupement azoté en 2 du cycle indoloquinolizine.

La présente invention a pour objet des composés de formule

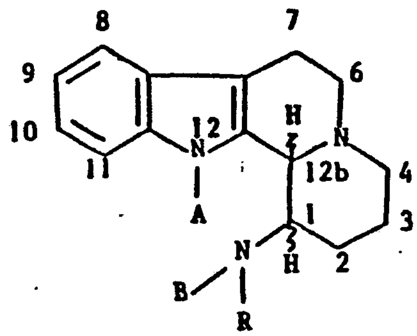

(I)

dans laquelle

R représente un atome d'hydrogène, un groupe alkyle en $C_1$-$C_4$, un groupe alcoxy ($C_1$-$C_4$) carbonyle ou un groupe benzoyle,

A représente un atome d'hydrogène,

B représente un atome d'hydrogène ou un groupe alkyle en $C_1$-$C_4$,

ou bien A et B forment ensemble un groupe de formule

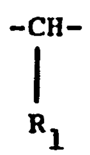 , $-CO-$, $-CH_2-CH_2-$ , ou $-CH_2CO-$,

$R_1$ représentant un atome d'hydrogène ou un groupe alcoxy ($C_1$-$C_4$) carbonyle, à la condition que lorsque B représente un atome d'hydrogène, R ne représente pas un groupe benzoyle, ainsi que leurs sels d'addition avec des acides pharmaceutiquement acceptables.

Les sels d'addition peuvent être formée par exemple avec les acides chlorhydrique, sulfurique, nitrique, phosphorique, méthane sulfonique, maléique, formique, fumarique, acétique, succinique, lactique, citrique, tartrique, glutamique, malique et les sels métalliques acides tels que l'orthosphosphate disodique et le sulfate monopotassique.

Les atomes d'hydrogène liés aux atomes de carbone 1 et 12b peuvent être soit en cis, soit en trans, et, pour un même composé, chacune de ces deux structures comprend deux antipodes optiques. La présente invention

englobe aussi bien les racémiques que les isomères optiques.

Les composés préférés sont ceux qui ont la structure trans.

Les composés de formule I peuvent être obtenus de la façon suivante:

a) on effectue une nitrosation d'une hexahydro-2,3,4,6,7,12 indolo [2,3-a] quinolizine de formule

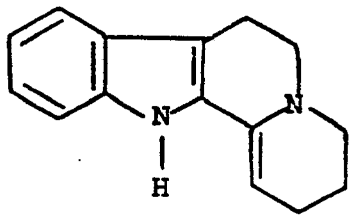

(II)

obtenant ainsi un dérivé nitroso de formule

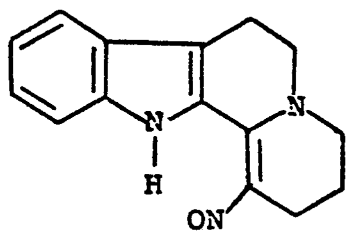

(III)

b) on traite ce dérivé nitroso en milieu acide fort obtenant ainsi un dérivé hydroxyimino insaturé de formule

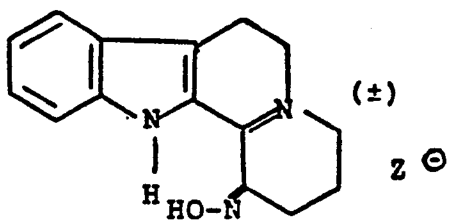

(IV)

Z⊖ représentent un reste d'acide fort.

c) on réduit ce dérivé hydroxyimino insaturé

- soit par le borohydrure de sodium en solution alcoolique avec réduction ultérieure du composé ainsi obtenu de formule

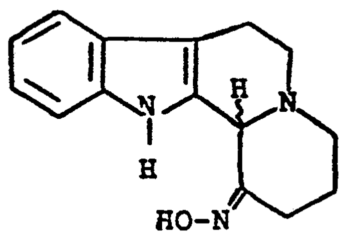

(V)

par l'hydrure de lithium et d'aluminium ou l'hydrure de bis (2-méthoxyéthoxy) aluminium sodium en solution dans un éther, obtenant ainsi un composé de formule Ia, de structure trans,

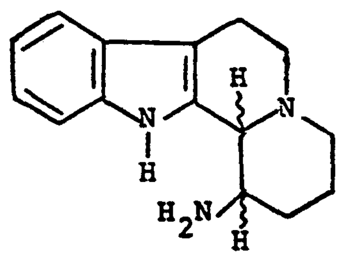

(Ia)

- soit par hydrogénation catalytique, obtenant ainsi un composé de formule Ia de structure cis, et

d) on transforme le composé Ia en autres composés de formule I par des réactions classiques, notamment des réactions d'acylation, d'acylation et de réduction et/ou d'alkylation.

Pour les réactions d'alkylation, on pourra se reporter à l'article "The Chemistry of the amino group" de Brian C. Challis et A.R. Butler (Edit. Saul Pataï - Interscience Publishers 1968).

Certains composés de formule I peuvent être obtenus soit par des réactions classiques d'alkylation, soit par une réaction d'acylation suivie d'une réaction de réduction.

Ainsi la monoalkylation des composés Ia peut être obtenue soit par conversion en amide ou en carbamate, suivie d'une réduction (voir exemple J.Org. Chem. 1965, 30, 2483), soit selon la séquence classique comportant une protection préalable, une alkylation et une déprotection (voir par exemple J. Chem. Soc. Serie C, 1969, 2223 ou J. Am. Chem. Soc. 1956, 78, 4778).

L'alkylation de l'amine secondaire ainsi obtenue peut être effectuée soit par alkylation directe, soit par conversion en amide ou carbamate suivie d'une réduction.

Les composés de formule I dans lesquels les groupes A et B sont liés ensemble peuvent être obtenus à partir des composés Ia par des réactions de cyclisation entrant dans le cadre général des réactions d'acylation, d'acylation/réduction et d'alkylation.

Ainsi, les composés de formule I dans lesquels A et B forment un pont

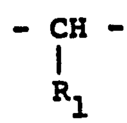

peuvent être obtenus par réaction des composés Ia avec un aldéhyde convenablement substitué en milieu neutre ou acide.

Les composés de formule I dans lesquels A et B forment un pont

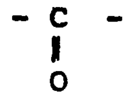

peuvent être obtenus par conversion en carbamate des composés Ia avec cyclisation ultérieure.

Les composés de formule I dans lesquels les groupes A et B forment un pont - $CH_2$ - CO - peuvent être obtenus par réaction des composés Ia avec des halogénures d'acides α-halogénés, notamment avec catalyse par transfert de phase.

Quant aux composés de formule I dans lesquels A et B forment un pont -$CH_2$ - $CH_2$ -, ils peuvent être obtenus par réaction des composés Ia avec des réactifs bifonctionnels du type α-diesters ou α-dicarbonylés.

Ils peuvent également être obtenus par réduction des dérivés carbonylés correspondants.

Dans les exemples qui suivent, les exemples 1 à 3 illustrent la préparation des produits intermédiaires, et les exemples 4 à 23 illustrent la préparation des composés de formule 1.

**Exemple de réference**

( ± ) Benzamido-1 octahydro-1,2,3,4,6,7,12,12b indolo [2,3-a] quinolizine.

2 g de ( ± ) amino-1 octahydro-1,2,3,4,6,7,12,12b indolo [2,3-a] quinolizine (exemple 4) sont dissous dans 100 ml de chlorure de méthylène en présence de 1,15 ml de triéthylamine. On ajoute alors 0,95 ml de chlorure de benzoyle et on laisse en contact 5 minutes. Le solvant est évaporé et le résidu obtenu dissous dans le chlorure de méthylène est chromatographié sur silice. L'élution est réalisée selon l'exemple 4. On récupère 2,4 g de benzamide. (Rt = 84%).

Préparation du fumarate

On solubilise l'amide dans le méthanol (0,01 mole) et ajoute l'acide fumarique (0,01 mole) en solution dans le méthanol.

On concentre la solution et laisse cristalliser.

F = 245°C

**Exemple 1**

Nitroso-1 hexahydro-2,3,4,6,7,12 indolo [2,3-a] quinolizine (Formule III).

A la température ambiante, on ajoute 150 ml de nitrite d'isoamyle à une suspension de 220 g d'hexahydro-2,3,4,6,7,12 indolo [2,3-a] quinolizine préparée selon la méthode décrite par R.N. Schutet T.J. Leipzig (J. Het. Chem. 1966, 3, 101) dans 1,2 l de méthanol. Après 16 h de repos, les cristaux sombres formés sont recueillis par filtration, rincés et séchés. On obtient 210 g de produit (Rt = 83 %).

Le produit cristallise avec 1/2 molécule de méthanol.

F = 195°C

**Exemple 2**

Chlorure d'hydroxyimino-1 heptahydro-1,2,3,4,6,7,12 indolo [2,3-a] quinolizinium (Formule IV).

A une suspension de 210 g de nitroso-1 hexahydro-2,3,4,6,7,12 indolo [2,3-a] quinolizine (exemple 1) dans 1,5 l de méthanol, maintenue au voisinage de la température de reflux, on ajoute lentement 120 ml d'acide chlorhydrique concentré. Le mélange est alors laissé 16 h en glacière. Par filtration 120 g de cristaux oranges sont recueillis (Rt = 79 %).

Le produit cristallise avec 2/3 de molécule d'eau.

F = 215°C

**Exemple 3**

( ± ) Hydroxyimino-1 octahydro-1,2,3,4,6,7,12,12b indolo [2,3-a] quinolizine (Formule V).

A 8,7 g de chlorure d'hydroxyimino-1 heptahydro-1,2,3,4,6,7,12 indolo [2,3-a] quinolizinium (exemple 2) en solution dans 200 ml de méthanol, on ajoute à température ambiante et par petites quantités et sous agitation du borohydrure de sodium, jusqu'à décoloration totale de la solution orange. Le mélange est alors jeté dans de l'eau et extrait par du chlorure de méthylène. La phase organique est lavée avec de l'eau, déshydratée sur

sulfate de sodium anhydre et évaporée. On récupère 7,5 g d'oxime (Rt = 98%).

Préparation de l'hémifumarate

On solubilise l'oxime (0,02 mole) dans le méthanol et on ajoute de l'acide fumarique (0,01 mole) en solution dans le méthanol. On concentre la solution et laisse cristalliser.

F = 192°C

**Exemple 4**

(±) Amino-1octahydro-1,2,3,4,6,7,12,12b indolo [2,3-a] quinolizine.

On met en suspension, à température ambiante, 14 g d'hydrure de lithium et d'aluminium dans 500 ml de tétrahydrofuranne, on ajoute alors goutte à goutte, en une heure, une solution de 50 g de (±)hydroxyimino-1 octahydro-1,2,3,4,6,7,12,12b indolo [2,3-a] quinolizine (exemple 3) dans 500 ml de tétrahydrofuranne. Sous forte agitation, l'excès d'hydrure est détruit par addition lente d'une solution saturée de sulfate de sodium. On porte à reflux 1/4 d'heure. Le précipité formé est filtré, puis rincé avec du chlorure de méthylène. Le filtrat est jeté dans de l'eau et est extrait plusieurs fois par du chlorure de méthylène. Après sèchage et évaporation de la phase organique, on obtient un résidu (43 g).

Ce résidu dissous dans du chlorure de méthylène est chromatographié sur une colonne de silice. On élue avec des solutions de chlorure de méthylène contenant des quantités croissantes de méthanol (0 à 5 %). Ce fractionnement fournit 15 g d'amine pure (21 %).

Préparation du difumarate

On solubilise l'amine (0,01 mole) dans le méthanol et on ajoute l'acide fumarique (0,02 mole) en solution dans le méthanol. On concentre la solution et laisse cristalliser.

F = 178°C

**Exemple 5**

(±) Iminométhano-[1,12] octahydro-1,2,3,4,6,7,12,12b indolo [2,3-a] quinolizine.

A 2,5 g de (±) amino-1 octahy dro-1,2,3,4,6,7,12,12b indolo [2,3-a] quinolizine (exemple 4) en solution dans 40 ml de tétrahydrofuranne, on ajoute 1 ml d'acide acétique et 1 ml dé formol à 30 % dans l'eau. La solution est portée au reflux durant une heure. Après refroidissement, le milieu réactionnel est versé dans de l'eau alcaline et extrait par du chlorure de méthylène. Après traitement habituel de la phase organique, on obtient 2,5 g d'un résidu, qui chromatographié sur silice selon l'exemple 4, fournit 2 g de produit pur (76 %).

Préparation du dichlorhydrate

On solubilise l'amine dans le méthanol (0,01 mole) et ajoute de l'acide chlorhydrique (0,02 mole) en solution méthanolique. On concentre et le dichlorhydrate cristallise avec une molécule d'eau.

F = 250°C (décomposition)

**Exemple 6**

(±) Ethoxycarbonyl-14 iminométhano- [1,12] -octahydro-1,2,3,4,6,7,12,12b indolo [2,3-a] quinolizine.

A température ambiante, on ajoute sous agitation 3 ml de chloroformiate d'éthyle à une solution de 3,6 g de (±) iminométhano- [1,12] octahydro-1,2,3,4,6,7,12,12b indolo [2,3-a] quinolizine (exemple 5) dans 100 ml de chlorure de méthylène. La phase chlorométhylénique est lavée avec de l'eau alcaline, séchée, puis évaporée. On obtient 4 g d'uréthanne (84 %).

**Exemple 6A**

Préparation du fumarate du composé de l'exemple 6

Il est préparé comme décrit à l'exemple de référence mentionné au début de la partie expérimentale.

F = 195-196°C

**Exemple 6B**

Préparation du méthanesulfonate du composé de l'exemple 6

La solution aqueuse du méthanesulfonate est lyophilisée.

Point de liquéfaction : 228-231° C

**Exemple 7**

( ± ) Benzoyl-14 iminométhano- [1,12] octahydro-1,2,3,4,6,7,12 12b indolo [2,3-a] quinolizine.

**Exemple 8**

( ± ) Oxo-14 iminoéthano-[1,12] octahydro-1,2,3,4,6,7,12,12b indolo [2,3-a] quinolizine.

On ajoute goutte à goutte, à température ordinaire 4 ml de bromure d'α-bromoacétyle à une solution de 10 g de ( ± ) amino-1 octahydro-1,2,3,4,6,7,12,12b indolo [2,3-a] quinolizine (exemple 4) dans 250 ml de chlorure de méthylène, sous forte agitation. Après 10 minutes, on ajoute 100 ml de lessive de soude (33 %) et 0,2 g d'hydrogénosulfate de tétrabutylammonium, et on laisse 1/2 heure sous forte agitation. Dans le milieu réactionnel laissé au repos, apparaît un précipité blanc dans la phase aqueuse. Il est recueilli par filtration et bien lavé avec de l'eau. Après séchage, on obtient 4 g de produit (38 %).

Préparation du fumarate

Il est préparé comme à l'exemple de référence.

Le fumarate cristallise avec une molécule d'eau.

F > 250° C

**Exemple 9**

( ± ) Iminoéthano-[1,12] octahydro-1,2,3,4,6,7,12,12b indolo [2,3-a] quinolizine.

3 g de ( ± ) oxo-14 iminoéthano [1,12] octahydro-1,2,3,4,6,7,12,12b indolo [2,3-a] quinolizine (exemple ) sont mis en suspension dans 250 ml de tétrahydrofuranne. On ajoute par petites quantités un excès d'hydrure de lithium et d'aluminium. Le mélange est porté 3 h à reflux, puis refroidi. 80 ml d'une solution saturée de sulfate de sodium sont alors ajoutés, sous forte agitation, avec précaution pour détruire l'excès d'hydrure. On porte de nouveau à reflux 1/4 d'heure, on refroidit et on filtre. Le précipité est rincé avec du chlorure de méthylène. Après traitement habituel de la phase organique, on récupère 2,3 g de produit (80 %).

Préparation du dichlorhydrate

Il est préparé comme à l'exemple 5.

F > 250° C

**Exemple 10**

( ± ) Amino-1 octahydro-1,2,3,4,6,7,12,12b indolo [2,3-a] quinolizine.

30 g de chlorure d'hydroxyimino-1 heptahydro-1,2,3,4,6,7,12 indolo [2,3-a] quinolizinium (exemple 2) en solution dans 300 ml d'acide acétique sont mis dans un appareil à hydrogéner en présence de 6 g de charbon palladié à 5 %, sous pression de 200 bars à température ambiante.

Quand l'absorption d'hydrogène est terminée le mélange est filtré. On rince plusieurs fois le catalyseur avec un mélange acide acétique/méthanol 50/50. Le filtrat est jeté dans de l'eau alcaline glacée et extrait par du chlorure de méthylène.

Après séchage et évaporation de la phase organique, on obtient 25 g de produit brut qui, chromatographié en solution dans le chlorure de méthylène sur une colonne d'alumine, fournit à l'élution par du chlorure de méthylène contenant des quantités croissantes de méthanol (0 à 5 %) 17,5 g d'amine pure (73 %).

Préparation du difumarate

Il est préparé selon l'exemple 4.

F = 115° C (décomposition)

**Exemple 11**

( ± ) Iminométhano-[1,12] octahydro-1,2,3,4,6,7,12,12b indolo [2,3-a] quinolizine.

On effectue une cyclisation dans des conditions analogues à celles de l'exemple 5.

**Exemple 12**

( ± ) Méthoxycarbonyl-13 iminométhano- [1,12] octahydro-1,2,3,4,6,7,12,12b indolo [2,3-a] quinolizine.

6 g de ( ± ) amino-1 octahydro-1,2,3,4,6,7,12,12b indolo [2,3-a] quinolizine (exemple 10) sont dissous dans 60 ml de tétrahydrofuranne et 20 ml d'acide acétique. On ajoute rapidement 33 ml de diméthoxyacétate de méthyle et on porte à reflux pendant 6 heures. Puis le mélange est jeté dans de l'eau alcaline et extrait plusieurs fois par le chlorure de méthylène. Après traitement habituel de la phase chlorométhylénique, le résidu chromatographié sur silice comme à l'exemple 4 fournit 2,55 g du produit (34 %).

Préparation de l'hémifurate

Il est préparé selon l'exemple 3.

F = 187°C

**Example 13**

( ± ) Ethoxycarbonylamino-1 octhaydro-1,2,3,4,6,7,12,12b indolo [2,3-a] quinolizine.

La réaction s'effectue dans des conditions analogues à celles de l'exemple 6.

**Exemple 14**

( ± ) Méthylamino-1 octahydro-1,2,3,4,6,7,12,12b indolo [2,3-a] quinolizine.

A 4,94 g de ( ± ) éthoxycarbonylamino-1 octahydro-1,2,3,4,6,7,12,12b indolo [2,3-a] quinolizine (exemple 13) dissous dans 100 ml de tétrahydrofuranne, on ajoute sous agitation un excès d'hydrure de lithium-aluminium par petites quantités. Lorsque l'addition est terminée, la suspension est alors portée à reflux durant 2 heures. Après refroidissement, l'excès d'hydrure est éliminé par une addition d'une solution saturée de sulfate de sodium. Le mélange est porté à reflux durant 15 minutes. On filtre et rince plusieurs fois avec du chlorure de méthylène. Le filtrat est jeté dans l'eau et extrait plusieurs fois par le chlorure de methylène. Après traitement habituel de la phase organique, on obtient 3,9 g de produit qui est chromatographié sur silice (comme à l'exemple 4).

Préparation du disulfate

Il est préparé selon l'exemple 11.

Le disulfate cristallise avec une 1/2 molécule d'eau.

F = 175°C (décomposition)

**Exemples 15 à 19**

Les diverses réactions s'effectuent dans des conditions analogues à celles des exemples ci-dessus.

**Exemple 20**

( ± ) Oxo-13 iminométhano- 1,12 octahydro-1,2,3,4,6,7,12,12b indolo [2,3-a] quinolizine.

4 g d'éthoxycarbonylamino-1 octahydro-1,2,3,4,6,7,12,12b indolo [1,3-a] quinolizine sont dissous dans 50 ml de tétrahydrofuranne. On ajoute par petites quantités 1 g d'hydrure de sodium (en suspension dans l'huile à 50 %) et on porte le mélange au reflux. Au bout d'une heure la réaction étant complète, l'excès d'hydrure est éliminé par une solution saturée de sulfate de sodium. On jette alors dans de l'eau acide et on extrait par de l'éther. La solution aqueuse alcalinisée est extraite par du dichlorométhane. La phase organique lavée puis séchée et évaporée, fournit 3,13 g de produit cyclisé (92,3 %).

Préparation du chlorhydrate.

Il est préparé selon l'exemple 7.

Le chlorhydrate cristallise avec 1/2 molécule d'eau.

F = 241-244°C

**Exemple 21**

( ± ) N-méthyl iminométhano -[ 1,12] octahydro-1,2,3,4,6,7,12,12b indolo [2,3-a] quinolizine.

La réaction s'effectue dans des conditions analogues à celles de l'exemple 14.

**Exemple 22**

( ± ) Oxo-13 iminométhano- [1,12] octahydro-1,2,3,4,6,7,12,12b indolo [2,3-a] quinolizine.

a) Dans 100 ml de dichlorométhane, on dissout 5 g de ( ± ) amino-1 octhaydro-1,2,3,4,6,7,12,12b indolo [2,3-a] quinolizine (exemple 4) et 3 ml de triéthylamine. Le mélange étant maintenu à 0° C, on ajoute goutte à goutte 2 ml de chloroformiate d'éthyle. On laisse le mélange maintenu sous agitation revenir à la température ordinaire pendant une heure. Après addition de 1 ml de chloroformiate d'éthyle, l'agitation est poursuivie pendant une heure supplémentaire.

La phase chlorométhylénique est alors lavée avec de l'eau alcaline, séchée puis évaporée. On isole 5 g de ( ± ) éthoxy-carbonylamino-1 octahydro-1,2,3,4,6,7,12,12b indolo [2,3-a] quinolizine (77 %).

b) 5 g de ( ± ) éthoxycarbonylamino-1 octahydro-1,2,3,4,6,7,12,12b indolo[2,3-a] quinolizine précédemment préparés sont mis en suspension dans 100 ml de tétrahydrofuranne. Par petites quantités, on ajoute de l'hydrure de sodium (en suspension dans de l'huile à 50 %) au mélange porté au reflux, jusqu'à ce qu'un nouveau produit apparaisse en chromatographie couche mince.

La réaction terminée, on détruit l'hydrure en excès par du méthanol et on jette le mélange dans de l'eau acide. Après lavage avec de l'éther, la solution aqueuse est alcalinisée et extraite par du dichlorométhane. Le traitement habituel de la phase organique permet d'isoler un résidu qui après chromatographie sur colonne de silice (comme à l'exemple 4) fournit 2 g de produit ( 45 %).

Préparation du sulfate

Il est préparé selon l'exemple 15.

Le sulfate cristallise avec 1/2 molécule d'eau.

F = 265-268° C

**Exemple 23**

( ± ) N-méthyl oxo-13 iminométhano- [1,12] octahydro-1,2,3,4,6,7,12,12b indolo [2,3-a] quinolizine.

Partant du composé obtenu selon l'exemple 24a, on effectue une réduction selon l'exemple 14, une éthoxycarbonylation selon l'exemple 6 et une cyclisation selon l'exemple 20.

Le tableau ci-après résume les structures et caractéristiques physiques des composés des exemples. Il précise également la position relative des atomes d'hydrogène liés aux atomes de carbone 1 et 12b.

TABLEAU

5

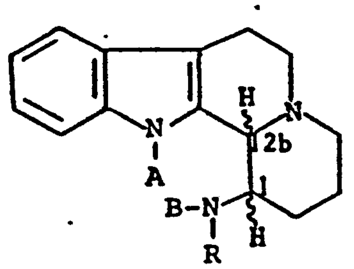

(±)

| Ex | H-1/H-12b | A | B | R | Sel | F(°C) |
|---|---|---|---|---|---|---|
| 4 | trans | H | H | H | difumarate | 178 |
| 5 | trans | $-CH_2-$ | | H | dichlorhydrate | 250 (d) |
| 6 | trans | $-CH_2-$ | | $C_2H_5OCO-$ | fumarate<br>mésylate | 195-6<br>228-31 |
| 7 | trans | $-CH_2-$ | | $C_6H_5CO-$ | chlorhydrate | 225 (d) |

TABLEAU (suite)

| Ex | H-1/H-12b | A | B | R | Sel | F(°C) |
|---|---|---|---|---|---|---|
| 8 | trans | $-CH_2-CO-$ | | H | fumarate | >250 |
| 9 | trans | $-CH_2-CH_2-$ | | H | dichlorhydrate | >250 |
| 10 | cis | H | H | H | difumarate | 115 (d) |
| 11 | cis | $-CH_2-$ | | H | disulfate | 182-5 |
| 12 | cis | $-CH(COOCH_3)-$ | | H | hémifumarate | 187 |
| 13 | cis | H | H | $C_2H_5OCO-$ | chlorhydrate | 260 (d) |
| 14 | cis | H | $CH_3$ | H | disulfate | 175 (d) |
| 15 | cis | H | $CH_3$ | $C_6H_5CO-$ | sulfate | 192-4 |
| 16 | cis | $-CH_2-CO-$ | | H | hémifumarate<br>chlorhydrate | 263 (d)<br>>280 |
| 17 | cis | $-CH_2-CO-$ | | $CH_3$ | fumarate | 247-52 |
| 18 | cis | $-CH_2-CH_2-$ | | H | fumarate | 220 |
| 19 | cis | $-CH_2-CH_2-$ | | $C_6H_5CO-$ | chlorhydrate | >280 |
| 20 | cis | -CO- | | H | chlorhydrate | 241-4 |
| 21 | trans | $-CH_2-$ | | $CH_3$ | dichlorhydrate | > 250 |
| 22 | trans | -CO- | | H | sulfate | 265-8 |
| 23 | trans | -CO- | | $CH_3$ | sulfate | > 250 |

(d) : fusion avec décomposition

Les composés de formule I et leurs sels d'addition avec des acides pharmaceutiquement acceptables présentent des propriétés pharmacologiques intéressantes et notamnent des propriétés antianoxiques et/ou vasodilatatrices prolongées.

Ils présentent en outre une toxicité qui n'apparait qu'à des doses très supérieures aux doses pharmacologiquement actives ce qui permet leur utilisation en thérapeutique.

On donne ci-après des résultats des études toxicologigues et pharmacologiques qui mettent en évidence ces propriétés.

**I - Toxicité aiguë chez la souris**

Les composés sont administrés par voie orale à doses croissantes (en progression arithmétique ou géométrique) à des lots de 3 à 5 souris femelles Swiss d'un poids moyen de 25 g.

Pour cela ils sont mis en suspension dans le soluté de gomme arabique à 10 %.

Les $DL_{50}$ sont calculées par la méthode de BEHRENS (B) et KARBER (C.) (Arch. Exp. Path. Pharmakol. 1935, 177, 379-388).

Le nombre d'animaux survivants dans les différents lots est vérifié définitivement 15 jours après l'administration des composés.

Les $DL_{50}$ ainsi déterminées vont de 500 mg/kg jusqu'à plus de 1000 mg/kg.

**II - Mise en évidence d'un effet antianoxique et vasorégulateur cérébral : Epreuve d'hypoxie hypobare chez la souris**

On utilise des souris mâles Charles River d'un poids moyen de 25 g réparties en lots de 6 à 10 animaux. Les composés sont administrés par voie orale, en solution dans l'eau distillée ou en suspension dans le soluté de gomme arabique à 10 %, 30 minutes avant l'épreuve.

Le produit de référence est la vincamine solubilisée sous forme de tartrate et administrée à la dose de 200 mg/kg per os.

Les souris sont introduites par 3 ( 1 souris témoin, 1 souris vincamine, 1 souris traitée par le produit) en atmosphère appauvrie en oxygène par réalisation d'un vide partiel dans une enceinte hermétiquement close où la pression est amenée à 185 mm Hg en 40 secondes à l'aide d'une pompe à vide.

On mesure le temps de survie des souris en prenant comme signe de la mort l'arrêt respiratoire. Ce temps est augmenté par les agents capables de favoriser l'oxygénation tissulaire cérébrale.

La moyenne des temps de survie est déterminée pour l'ensemble des animaux témoins et pour chacun des lots traités. Les pourcentages d'augmentation du temps de survie par rapport aux valeurs observées chez les animaux témoins sont calculés.

L'analyse statistique des résultats est effectuée par le test non paramétrique de Mann et Whitney (les comparaisons sont réalisées par rapport aux témoins).

L'importance des effets antianoxiques des composés de formule I est appréciée par rapport à ceux obtenus avec la dose régulièrement active de 200 mg/kg per os de vincamine.

On constate ainsi gue, pour des doses de substances actives allant de 20 mg/kg à 100 mg/kg, les temps de survie des animaux d'essai sont du même ordre de grandeur, ou le plus souvent supérieurs, à ceux obtenus avec la vincamine à 200 mg/kg.

**III - Mise en évidence d'un effet vasodilatateur périphérique: Etude sur le système cardiovasculaire du chien anesthésié**

L'étude des composés de l'invention a été réalisée chez des chiens mâles bâtards d'un poids compris entre 16 et 28 kg, anesthésiés au pentobarbital (30 mg/kg I.V.), l'anesthésie étant entretenue au cours de l'essai par une perfusion lente discontinue de pentobarbital (2 à 5 mg/kg/ heure sous un volume de 1,2 à 3 ml).

Les animaux sont placés en respiration assistée.

Les effets vasodilatateurs périphériques sont recherchés par enregistrement des variations du débit moyen d'une artère fémorale.

Les composés solubilisés dans l'eau distillée, sont administrés par injection dans la veine saphène à des doses correspondant approximativement aux 1/800e, 1/400e, 1/200e, 1/100e, 1/50e, 1/20e et 1/10e de la $DL_{50}$ déterminée par voie intraveineuse chez la souris.

Les composés de formule I augmentent très nettement les débits vasculaires périphériques à partir de doses faibles, et se révèlent particulièrement intéressants par la durée de leurs effets.

Les composés de formule I et leurs sels d'addition avec des acides pharmaceutiguement acceptables sont utilisables en thérapeutique notamment dans le traitement des accidents vasculaires cérébraux, des insuffisances circulatoires cérébrales, des troubles circulatoires des extrémités, des arthériophaties des membres.

Ces composés et leurs sels d'addition avec des acides pharmaceutiquement acceptables peuvent être administrés à l'homme par voie orale, rectale ou parentérale, notamment en association avec un excipient pharmaceutiquement acceptable.

Ils peuvent être présentés sous forme de comprimés, gélules ou toute autre forme destinée à la voie orale, sous forme de suppositoires, sous forme de préparations pour les voies parentérales.

Les compositions peuvent être administrées à des doses unitaires comprises entre 1 et 50 mg de principe actif.

L'administtation chez l'homme peut être effectuée à des doses allant de 5 mg à 250 mg par jour.

**Revendications**

1. Composés sous forme de racémates ou isomères optiques, répondant à la formule

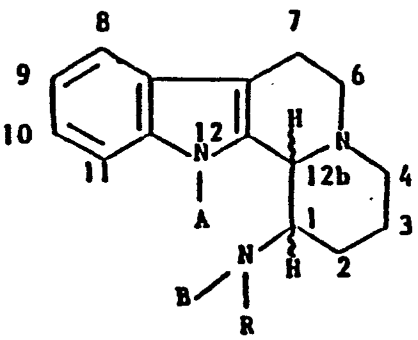

(I)

dans laquelle

R représente un atome d'hydrogène, un groupe alkyle en $C_1$-$C_4$, un groupe alcoxy ($C_1$-$C_4$) carbonyle ou un groupe benzoyle,

A représente un atome d'hydrogène,

B représente un atome d'hydrogène ou un groupe alkyle en $C_1$-$C_4$,

ou bien A et B forment ensemble un groupe de formule

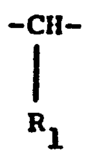 , $-CO-$, $-CH_2-CH_2-$ , ou $-CH_2CO-$,

$R_1$ représentant un atome d'hydrogène ou un groupe alcoxy ($C_1$-$C_4$) carbonyle, à la condition que lorsque B représente un atome d'hydrogène, R ne représente pas un groupe benzoyle, ainsi que leurs sels d'addition avec des acides pharmaceutiquement acceptables.

2. La (±)amino-1 octahydro-1,2,3,4,6,7,12,12b indolo [2,3-a] quinolizine de structure trans, ainsi que ses sels d'addition à des acides pharmaceutiquement acceptables.

3. La (±) oxo-13 iminométhano- [1,12] octahydro-1,2,3,4,6,7,12,12b indolo [2,3-a] quinolizine de structure trans, ainsi que ses sels d'addition à des acides pharmaceutiquement acceptables.

4. Procédé de préparation des composés de formule I tels que définis à la revendication 1, caractérisé en ce que

a) on effectue une nitrosation d'une hexahydro-2,3,4,6,7,12 indolo [2,3-a] quinolizine de formule

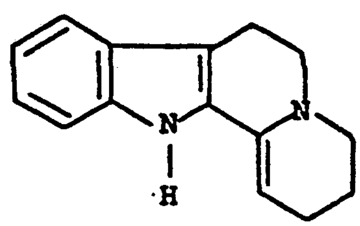

(II)

obtenant ainsi un dérivé nitroso de formule

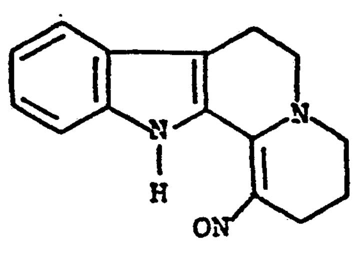

(III)

b) on traite ce dérivé nitroso en milieu acide fort obtenant ainsi un dérivé hydroxyimino insaturé de formule

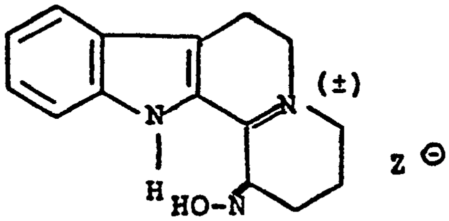

(IV)

$Z^{\ominus}$ représentent un reste d'acide fort,

c) on réduit ce dérivé hydroxyimino insaturé

- soit par le borohydrure de sodium en solution alcoolique avec réduction ultérieure du composé ainsi obtenu de formule

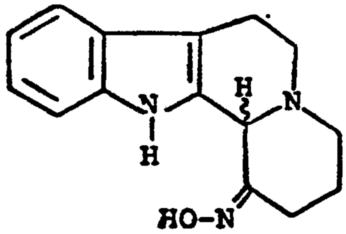

(V)

par l'hydrure de lithium et d'aluminium ou l'hydrure de bis (2-méthoxyéthoxy) aluminium sodium en solution dans un éther,

- soit par hydrogénation catalytique, obtenant ainsi un composé de formule

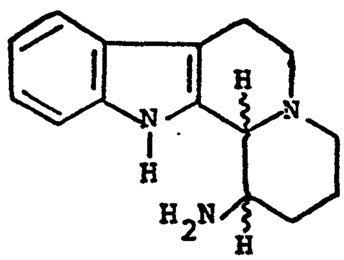

(Ia)

et

d) on transforme le composé Ia en autres composés de formule I par des réactions classiques.

5. Composition thérapeutique, caractérisée en ce qu'elle contient à titre de principe actif un composé selon l'une quelconque des revendications 1 à 3.

## Patentansprüche

1. Verbindungen in Form von Razematen oder optischen Isomeren der Formel

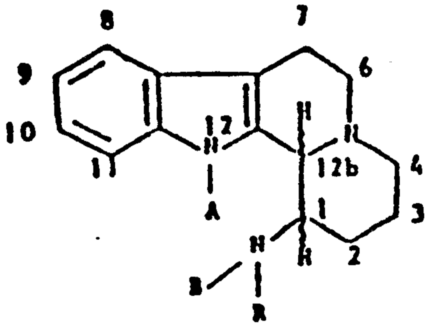

(I)

worin R ein Wasserstoffatom eine $C_1$-$C_4$-Alkylgruppe, eine Alkoxy ($C_1$-$C_4$)-carbonylgruppe oder eine Benzoylgruppe bedeutet,
A ein Wasserstoffatom bedeutet,
B ein Wasserstoffatom oder eine $C_1$-$C_4$-Alkylgruppe bedeutet,
oder A und B gemeinsam eine Gruppe der Formel

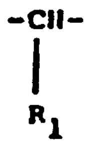 , $-CO-$, $-CH_2-CH_2-$, oder $-CH_2CO-$,

bilden, worin $R_1$ ein Wasserstoffatom oder eine Alkoxy-($C_1$-$C_4$)-carbonylgruppe bedeutet, unter der Voraussetzung, daß, wenn B ein Wasserstoffatom bedeutet, R keine Benzoylgruppe darstellt, sowie deren Additionssalze mit pharmazeutisch verträglichen Säuren.

2. (±)1-Amino-1,2,5,4,6,7,12,12b-octahydro-indolo-[2,3-a]-chinolizin mit trans-Struktur sowie dessen Additionssalze mit pharmazeutisch verträglichen Säuren.

3. (±)13-Oxo-iminomethano-[1,12]-1,2,5,4,6,7,12,12b-octahydro-indolo-[2,3-a] -chinolizin mit trans-Struktur sowie dessen Additionssalze mit pharmazeutisch verträglichen Säuren.

4. Verfahren zur Herstellung von Verbindungen der Formel (I) gemäß Anspruch 1, dadurch gekennzeichnet, daß man

a) eine Nitrosierung eines 2,3,4,6,7,12-Hexahydro-indolo-[2,3-a]-chinolizins der Formel

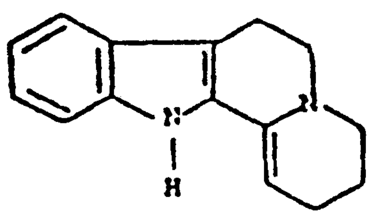

(II)

unter Erzielung eines Nitrosoderivats der Formel

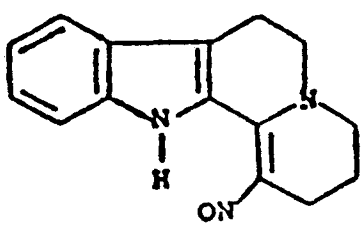

(III)

durchführt,

b) dieses Nitrosoderivat in stark saurem Milieu unter Erzielung eines ungesättigten Hydroxyiminoderivats der Formel

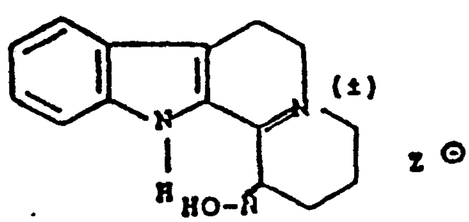

(IV)

worin $Z^{\ominus}$ einen Rest einer starken Säure bedeutet, behandelt,

c) dieses ungesättigte Hydroxyiminoderivat

- entweder mit Natriumborhydrid in alkoholischer Lösung unter späterer Reduktion der so erhaltenen Verbindung der Formel

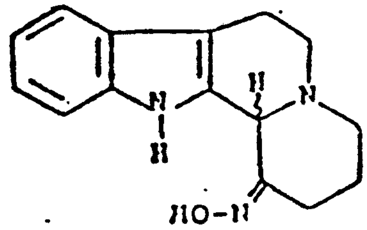

(V)

mit Lithiumaluminiumhydrid oder Bis-(2-methoxy-äthoxy)-aluminiumnatriumhydrid in ätherischer Lösung.
- oder durch katalytische Hydrierung unter Erzielung einer Verbindung der Formel

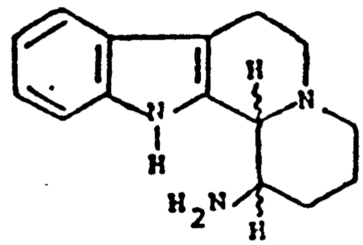

(Ia)

reduziert, und
d) die Verbindung (Ia) in weitere Verbindungen der Formel (I) nach klassischen Reaktionen überführt.
5. Therapeutische Zusammensetzung, dadurch gekennzeichnet, daß sie als Wirkstoff eine Verbindung gemäß einem der Ansprüche 1 bis 3 enthält.

**Claims**

1. Compounds in the form of racemates or optical isomers, answering to the formula

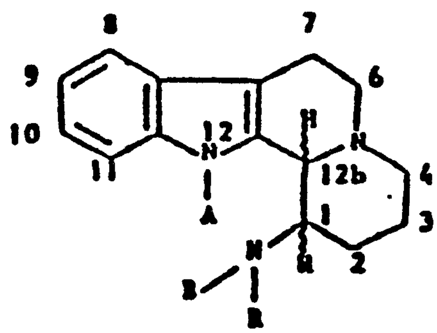

(I)

in which
R represents a hydrogen atom, an alkyl group in $C_1$-$C_4$, an alkoxy ($C_1$-$C_4$) carbonyl group or a benzoyl group,
A represents a hydrogen atom,
B represents a hydrogen atom or an alkyl group in $C_1$-$C_4$,
or A and B together form a group with the formula

$$\underset{\displaystyle R_1}{-\underset{|}{C}H-}, \; -CO-, \; -CH_2-CH_2-, \text{ or } -CH_2CO-,$$

$R_1$ representing a hydrogen atom or an alkoxy ($C_1$-$C_4$) carbonyl group, on condition that when B represents a hydrogen atom, R does not represent a benzoyl group, as well as their salts of addition with pharmaceutically acceptable acids.
2. (±)amino-1-octahydro-1,2,3,4,6,7,12,12b-indolo [2,3-a] quinolizine of trans structure, as well as its salts of addition with pharmaceutically acceptable acids.

3. (±)oxo-13 iminomethano-[1, 12]octahydro-1,2,3,4,6,7,12,12b-indolo [2,3-a]quinolizine of trans structure, as well as its salts of addition with pharmaceutically acceptable acids.

4. Preparation process for compounds with the formula (I) as defined in claim 1, characterized in that

a) a nitrosation of a hexahydro-2,3,4,6,7,12-indolo[2,3-a]quino-lizine with the formula

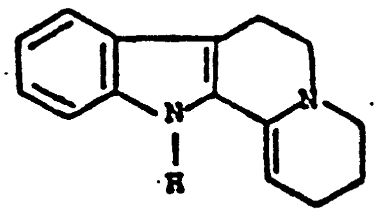

(II)

is carried out, thus obtaining a nitroso derivative with the formula

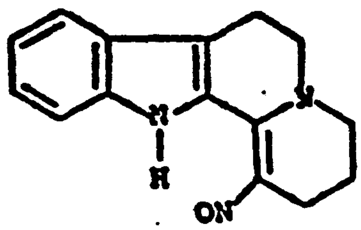

(III)

b) this nitroso derivative is treated in a strong acid medium thus obtaining an unsaturated hydroxyimino derivative with the formula

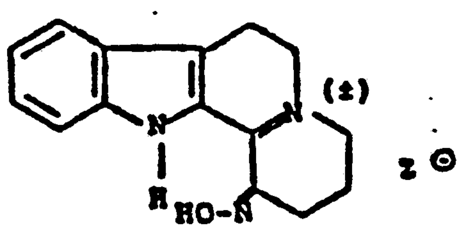

(IV)

$Z^{\ominus}$ representing a strong acid residue,

c) this unsaturated hydroxyimino derivative is reduced

- either by sodium borohydride in alcoholic solution with further reduction of the compound thus obtained with the formula

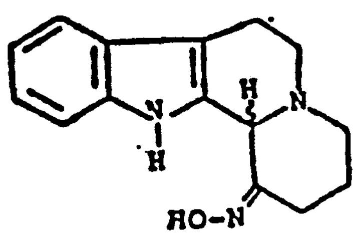

(V)

by lithium and aluminium hydride or bis(2-methoxyethoxy) aluminium sodium hydride in solution in an ether,

- or by catalytic hydrogenation, thus obtaining a compound with the formula

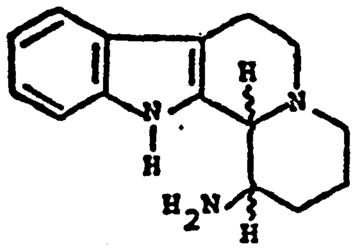

(Ia)

and

d) the compound (Ia) is converted into other compounds with the formula (I) by standard reactions.

5. Therapeutic composition, characterized in that it contains as active principle a compound according to any one of the claims 1 to 3.